# EUROPEAN PATENT SPECIFICATION

(11) **EP 2 400 930 B1**
(45) Date of publication and mention of the grant of the patent: **27.12.2017**
(21) Application number: 10746863.9
(22) Date of filing: 25.02.2010
(51) Int. Cl.: A61F 2/38, A61F 2/46, A61B 17/17, A61B 17/16, A61B 17/86

(54) **TROCHLEAR RESURFACING SYSTEM**
TROCHLEAR-OBERFLÄCHENERSATZSYSTEM
SYSTÈME DE RESURFAÇAGE

(30) Priority: 25.02.2009 US 155390 P
(43) Date of publication of application: 04.01.2012
(73) Proprietor: Arthrosurface Incorporated, Franklin, MA 02038 (US)
(72) Inventor: EK, Steven W., Bolton MA 01740 (US); SIKORA, George, Bridgewater Massachusetts 02324 (US)
(74) Representative: Lippert Stachow Patentanwälte Rechtsanwälte
(86) International application number: PCT/US2010/025464
(87) International publication number: WO 2010/099357

(56) References cited:
- EP-A2- 0 240 004
- EP-A2- 1 374 782
- US-A- 4 788 970
- US-A- 4 920 958
- US-A- 5 616 146
- US-A1- 2003 225 456
- US-A1- 2008 172 125
- US-B2- 6 984 248
- US-B2- 6 984 248
- US-B2- 7 290 347

## Description

### FIELD

This disclosure relates to devices and methods for the repair of defects that occur in articular cartilage on the surface of bones, particularly the knee.

### BACKGROUND

Articular cartilage, found at the ends of articulating bone in the body, is typically composed of hyaline cartilage, which has many unique properties that allow it to function effectively as a smooth and lubricious load-bearing surface. When injured, however, hyaline cartilage cells are not typically replaced by new hyaline cartilage cells. Healing is dependent upon the occurrence of bleeding from the underlying bone and formation of scar or reparative cartilage called fibrocartilage. While similar, fibrocartilage does not possess the same unique aspects of native hyaline cartilage and tends to be far less durable.

In some cases, it may be necessary or desirable to repair the damaged articular cartilage using an implant. While implants may be successfully used, the implant should have a shape substantially corresponding to the articular cartilage proximate the area where the implant is to be placed in order to maximize the patient's comfort, minimize damage to surrounding areas, and maximize the functional life of the implant. US 2008/0172125 A1 discloses a system for repairing a defect on an articular surface of a patient's bone, comprising a reamer having a shaft and a cutting surface on a cutting head, a guide block comprising a body configured to engage against said articular surface, a protrusion configured to be received in a bore formed in said articular surface along a reference axis, and an excision passageway along a working axis. With this system the reamer cannot be changed without taking the guide block off the articular surface.

### SUMMARY OF THE INVENTION

According to one aspect of the invention, there is provided a system for repairing a defect on an articular surface of a patient's bone according with claim 1.

Also disclosed is a method for preparing an implant site in bone. Said method comprises establishing a reference axis extending from said bone, creating a bore in said bone by reaming about said reference axis, securing a guide block about said articular surface, establishing a first working axis extending from said bone using said guide block, wherein said first working axis is displaced from said reference axis, and creating a first socket in said bone by reaming about said first working axis, wherein said first socket partially overlaps with said bore.

Said method may further be provided such that said guide block comprises a body including a base portion and sidewall portions having a generally arcuate shaped exterior surface generally configured to engage with the saddle portion and ridge portions of a patient's trochlear region, respectively.

Also, in said method, securing said guide block may comprise advancing a protrusion extending generally from a body of said guide into said bore.

Also, in said method, securing said guide block may comprise advancing at least one pin through a passageway in said body and into bone proximate to said trochlear region.

Further, said method may comprise establishing a second working axis extending from said bone using said guide block, wherein said first working axis is displaced from said reference axis, as well as creating a second socket in said bone by reaming about said first working axis, wherein said second socket partially overlaps with said bore and wherein said first and second sockets and said bore are generally aligned along an inferior-superior plane of said articular surface.

Further, said method may comprise advancing a reamer through a cavity extending through said body of said guide block after said guide block is secured to said articular surface, inserting a guide bushing into said cavity subsequent to advancing said reamer, said guide block comprising a first excision passageway configured to receive a shaft of said reamer along said first working axis, wherein a said radial cutter of said reamer is disposed adjacent to said articular surface, as well as rotating said reamer within said first excision passageway and advancing said radial cutter into said articular surface to form create said first socket.

### BRIEF DESCRIPTION OF THE DRAWINGS

Features and advantages of the present invention are set forth by description of embodiments consistent with the present invention, which description should be considered in conjunction with the accompanying drawings wherein:
**FIG. 1** is a schematic diagram illustrating an incision proximate the knee;
**FIG. 2** is a schematic diagram illustrating the femur;
**FIG. 3** is a perspective view of one embodiment of a drill guide consistent with the present disclosure;
**FIG. 4** is a perspective view of one embodiment of the drill guide on the articular surface to establish the reference axis consistent with the present disclosure;
**FIG. 5** is a perspective view of one embodiment of a pin and the articular surface consistent with the present disclosure;
**FIG. 6** is a perspective view of one embodiment of a contact probe disposed about the articular surface consistent with the present disclosure;
**FIG. 6A** is a close-up of region **6A** in **FIG. 6** consistent with the present disclosure;
**FIG. 7** is a perspective view of one embodiment of a contact probe along the inferior-superior and medial-lateral planes consistent with the present disclosure;
**FIG. 8** illustrates one embodiment of a sizing card consistent with the present disclosure;
**FIG. 9** is a perspective view of one embodiment of a surface reamer consistent with the present disclosure;
**FIG. 10** is a perspective view of one embodiment of a surface reamer aligned with a guide pin and a drill consistent with the present disclosure;
**FIG. 11** is a perspective side view of one embodiment of a guide block consistent with the present disclosure;
**FIG. 12** is a perspective view of one embodiment of a guide block and securing pins consistent with the present disclosure;
**FIG. 13** is a perspective view of one embodiment of a guide bushing consistent with the present disclosure;
**FIG. 14** is a perspective view of one embodiment of a guide block and a guide bushing received therein consistent with the present disclosure;
**FIG. 15** is a perspective view of one embodiment of an implant consistent with the present disclosure;
**FIG. 16** is another perspective view of one embodiment of the implant shown in **FIG. 15** consistent with the present disclosure;
**FIG. 17** is a top end perspective view of one embodiment of the implant shown in **FIG. 15** consistent with the present disclosure;
**FIG. 18** is a bottom end perspective view of one embodiment of the implant shown in **FIG. 15** consistent with the present disclosure; and
**FIG. 19** is a cross-sectional view of one embodiment of the guide block shown in **FIG. 11** consistent with the present disclosure.

### DETAILED DESCRIPTION

According to one embodiment, the present disclosure may feature a system for resurfacing at least a portion of an articular surface having a defect by replacing a portion of the articular surface with an implant. The implant may comprise a load bearing surface having a contour and/or shape substantially corresponding to the patient's original articular surface about the defect site which may be configured to engage an adjacent articular surface. The present disclosure will describe a system and method for replacing a portion of the articular surface of the trochlear region; however, it should be understood that the system and method according to the present disclosure may also be used to resurface articular surfaces other than the trochlear region.

As an initial matter, many of the devices described herein comprise cannulated components configured to be arranged over other components. The degree to which the cannulated passageway (i.e., internal diameter of the passageway/cavity) of a first component corresponds to the external diameter of the component over which it is being placed may be close enough to generally eliminate excessive movement. Excessive movement may be defined as an amount of movement that may result in misalignment of the implant relative to the articular surface.

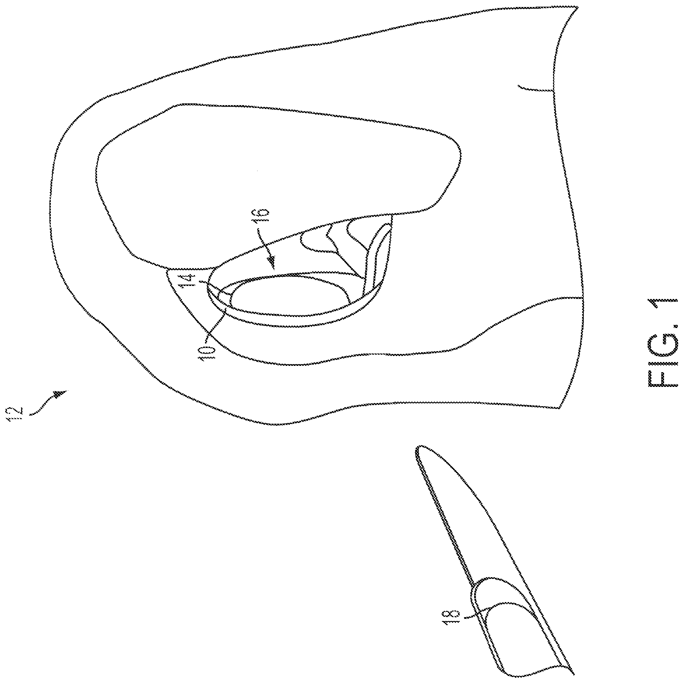

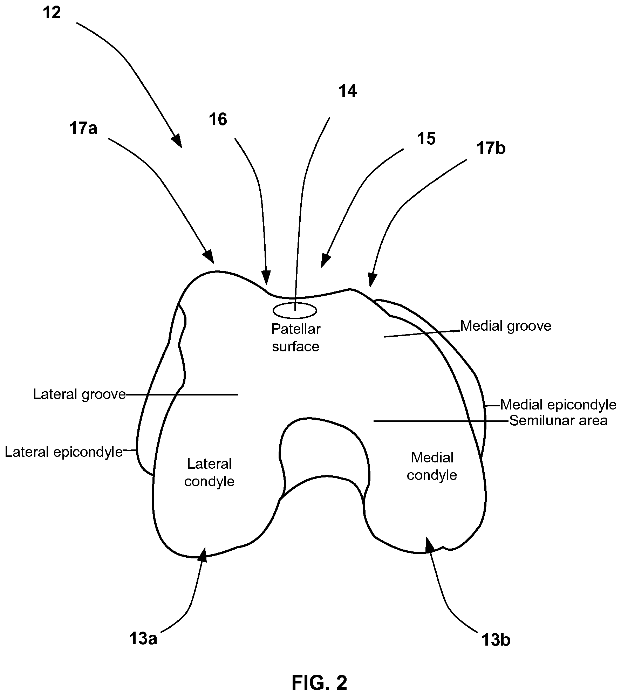

Turning now to **FIGS. 1** and **2****,** an incision 10 may be created proximate the patient's knee 12 (only the femur of which is illustrated for clarity) using a cutting instrument 18 (e.g., a surgical knife) to provide access to the defect 14 on the patient's articular surface 16, for example, as taught in U.S. Pat. No. 7,896,883 entitled FEMORAL CONDYLE RESURFACING SYSTEM AND METHOD. As generally illustrated in **FIG. 2****,** the defect 14 may be generally located within the trochlear region of the knee 12 generally between the lateral and medial condyles 13a, 13b. More specifically, the defect 14 may be generally located at a region that cooperates with a patellar (not shown for clarity).

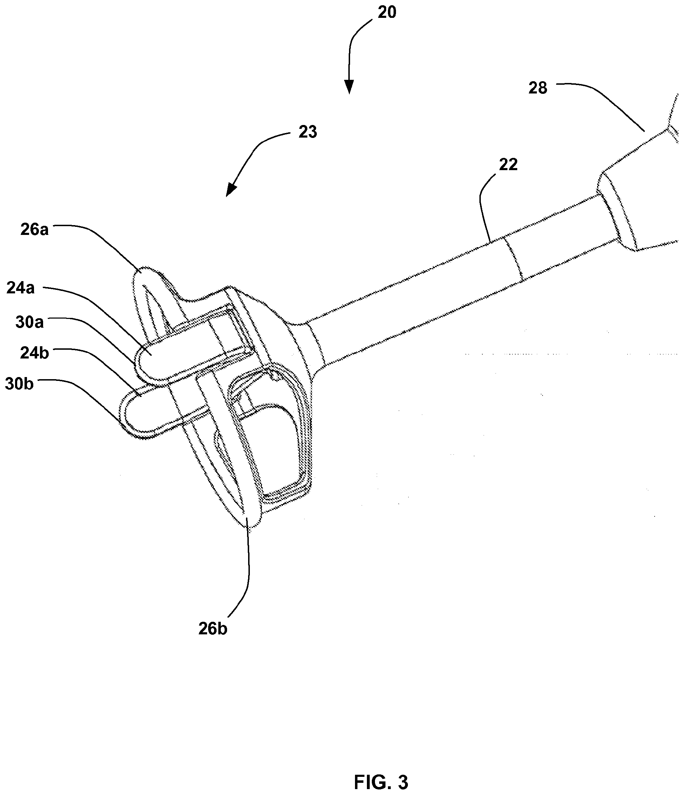

Once the incision is created, a drill guide 20, **FIG. 3****,** may be advanced against the articular surface 16, for example, in the general area of the trochlear region. The drill guide 20 may include a cannulated shaft 22, a proximal end 23 comprising a first and second groove contacting tip 24a, 24b configured to contact or engage with the articular surface 16 in the base or lower region 15 of the trochlear region (generally illustrated in **FIG. 1**). The first and second groove contacting tip 24a, 24b may optionally include a generally "C" like shape which may be fixedly coupled to the cannulated shaft 22 and may include a first and second tip 30a, 30b configured to contact the articular surface 16 at two different points generally along the inferior-superior plane.

The drill guide 20 may also include a first and second ridge contacting tip 26a, 26b configured to contact or engage with the articular surface 16 on the ridges 17a, 17b generally defined by the lateral and medial condyles (generally illustrated in **FIG. 1**). The first and second ridge contacting tips 26a, 26b may optionally include a generally arcuate shape extending generally radially outwardly and away from the cannulated shaft 22. The first and second ridge contacting tip 26a, 26b may also be moveably coupled to the cannulated shaft 22 and may be biased towards an extended position as generally illustrated in **FIG. 2** using a spring or the like (not shown). The first and second ridge contacting tip 26a, 26b may be configured to at least partially contact the articular surface 16 at two different points on the ridge generally along the medial-lateral plane.

Because the tips 24a, b and 26a, b are moveable with respect to each other, the drill guide 20 may be advanced against the articular surface 16 until a portion of the tips 24a, 24b contact the articular surface 16 generally along the inferior-superior plane of the articular surface 16 and the tips 26a, 26b contact the articular surface 16 generally along the medial-lateral (ML) plane of the articular surface 16. The four points of contact of the tips 24a, b and 26a, b of the drill guide 20 may be proximate, but generally not within, the defect site 14 and may be used to establish a reference axis extending generally approximately normal to the articular surface 16 about the defect site 14, for example, as generally described in U.S. Pat. No. 7, 896, 883. The four points of the drill guide 26a, 26b, 30a, and 30b may be configured asymmetrical to the axis of shaft 22 to create a repair site that would cover slightly more of the lateral facet of the trohclear groove.

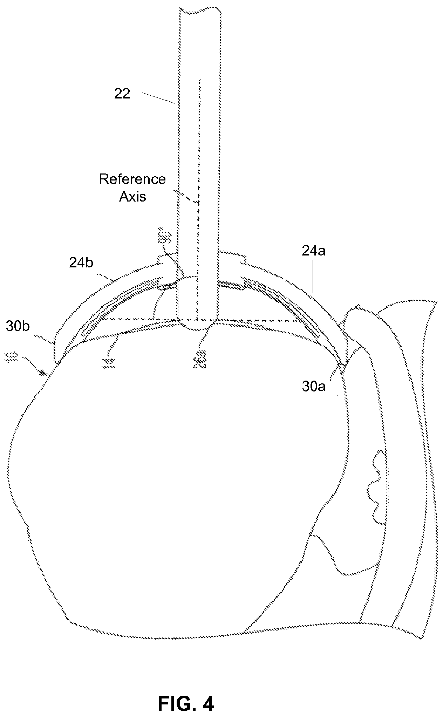

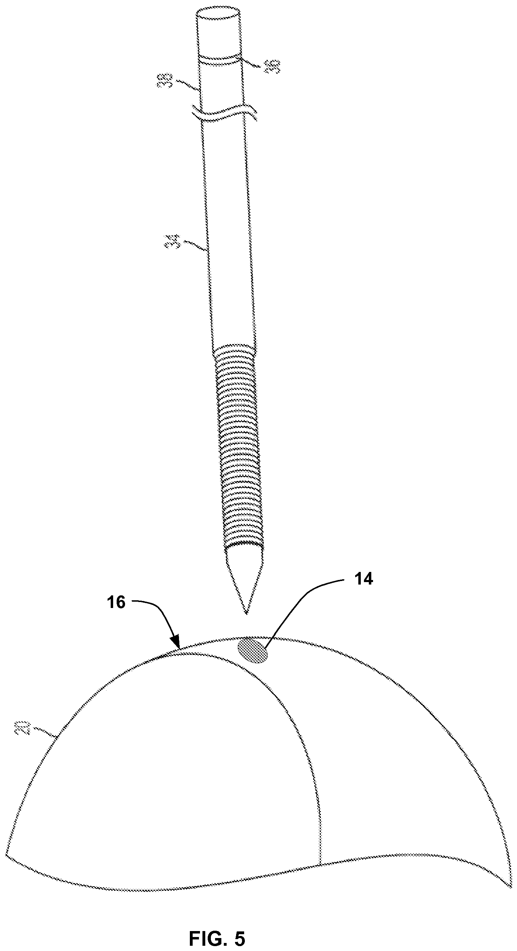

With the four points of the drill guide 20 against the articular surface 16, a threaded guide pin 34, **FIG. 5****,** may be advanced through the cannulated drill guide 20 along the reference axis and into the bone beneath the defect site 14, for example using a drill or the like. The guide pin 34 may include one or more indicia 36 (for example, but not limited to, laser markings or the like) on the shaft 38 of the guide pin 34 that may be used to control the depth of the guide pin 34 into the bone. By way of example, the indicia 36 on the guide pin 34 may be set relative to the length of the drill guide 20 such that the depth of the guide pin 34 is set when the indicia 36 is aligned with the distal end of the drill guide 20. Once the guide pin 34 is coupled to the bone, the drill and the drill guide 20 may be removed leaving just the guide pin 34 coupled to the bone and extending along the reference axis (i.e., substantially normal/perpendicular to the original articular surface about the defect site 14 as generally illustrated in **FIG. 4**)**.** It should be noted that the cannulated passageway of the drill guide 20 may have an internal diameter substantially corresponding to the outer diameter of the guide pin 34, for example, as generally described in U.S. Pat. No. 7, 896, 883.

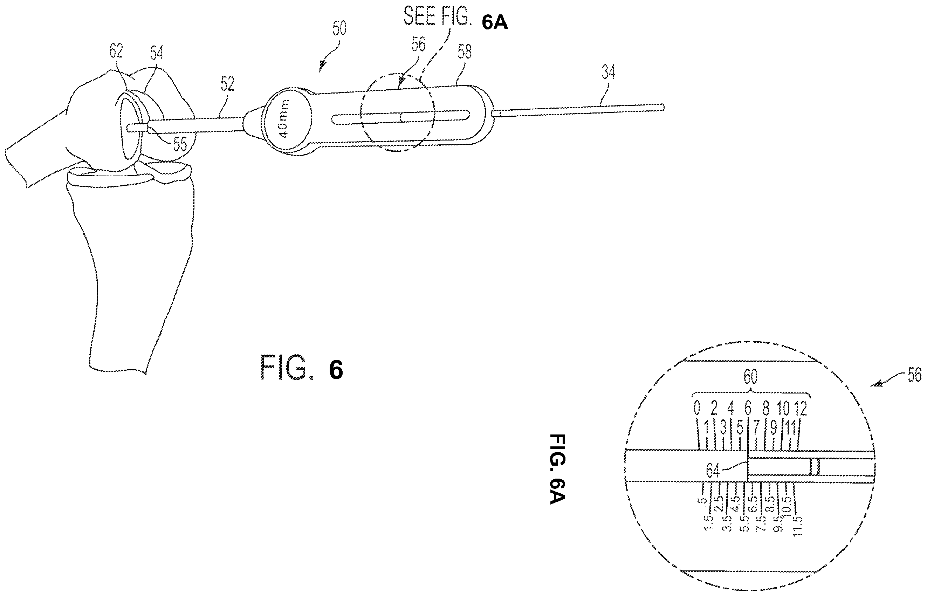

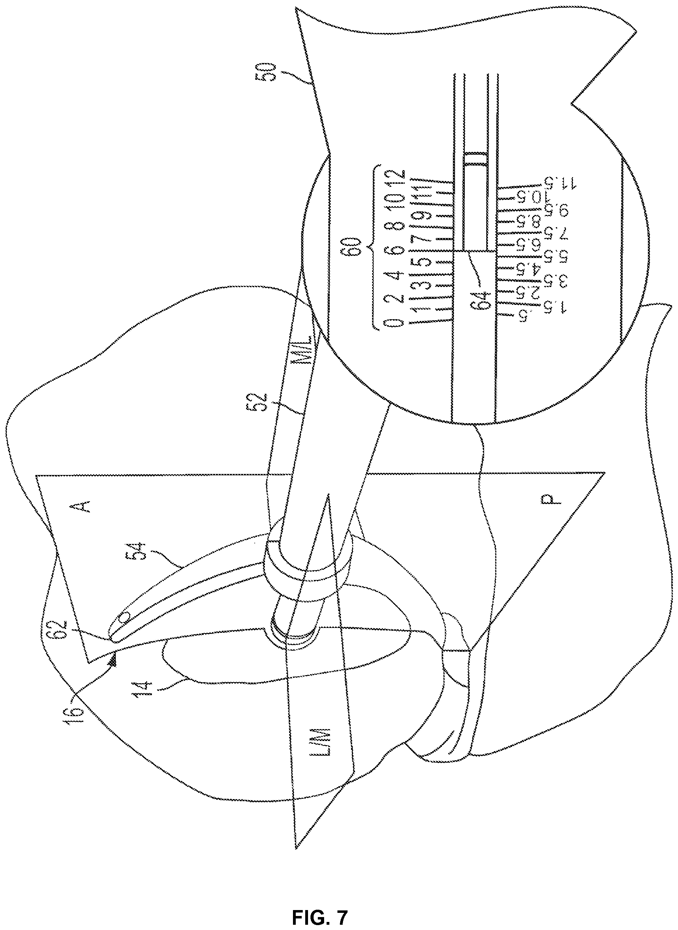

Next, measurements of the patient's articular surface 16 may be taken in order to determine the appropriate contour of the implant, **FIGS. 6-8****.** For example, one or more contact probes 50 may be advanced over the guide pin 34 established in the articular surface 16. The contact probe 50 may comprise a cannulated shaft 52 and an outrigger 54 extending radially outwardly and axially outwardly from a distal end of the cannulated shaft as generally taught in U.S. Pat. No. 7, 896, 883. A first and a second contact probe 50 may be provided having outriggers 54 extending radially outwardly at a two different distances. The distances of the outriggers 54 may be dependent upon the size of the implant to be delivered as well as the geometry of the defect site 14 and/or the articular surface 16.

The contact probe 50 may also include measuring indicia 60, which may optionally be disposed in a portion of a handle 58. The measuring indicia 60 may include a plurality of measurement markings indicating relative distances. In use, the contact probe 50 may be placed over the guide pin 34 such that the distal end 62 of the outrigger 54 contacts the articular surface 16. A measurement may be taken by based on the alignment of at least one marking 64 on the centering shaft (for example, the second end of the centering shaft) with the plurality of measurement markings 60.

A first (and optionally a second) measurement of the patient's articular surface 16 proximate the defect site 14 may be taken along the inferior-superior plane using the first contact probe 50 by placing the distal end 62 of the outrigger 54 against the patient's articular surface 16. In addition, a first (and optionally a second) measurement of the patient's articular surface 16 proximate the defect site 14 may be taken along the ML plane using the second contact probe 50 by placing the distal end 62 of the outrigger 54 against the patient's articular surface 17a, 17b. The size of the outriggers 54 may be selected based on the size of the defect site 14 such that the distal end 62 of the outrigger 54 contacts the articular surface 16 and not the defect site 14.

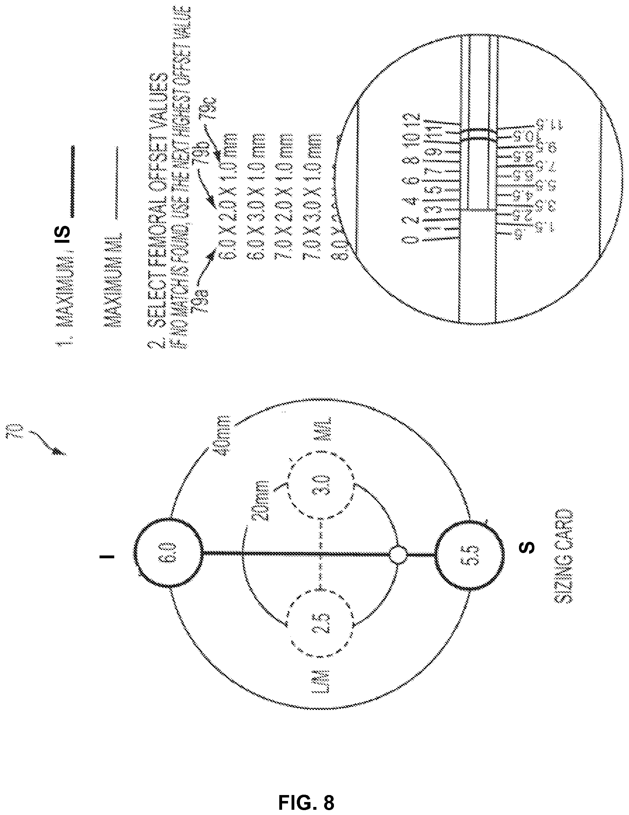

The measurements obtained from the contact probes may be recorded onto a sizing card 70, **FIG. 8****,** as generally taught in U.S. Pat. No. 7,896,883. The sizing card 70 may include an area graphically representing the inferior-superior and the ML planes. In particular, a first and a second query box may be provided to fill in the first and second inferior-superior measurements and a first and a second query box may be provided to fill in the first and second ML measurements. The query boxes may optionally be connected by a circle representing the size of the outrigger of the first contact probe while the other query boxes may optionally be connected by a circle representing the size of the outrigger of the second contact probe. The sizing card may also include additional query boxes provided to fill in the maximum values of the inferior-superior plane and the ML plane, respectively.

Based on the maximum values of the inferior-superior and ML plane in query boxes, the offset values of the implant and test implant may be determined. The surgeon may select from a set of implants having predetermined offset values. The values correspond to the inferior-superior measurement, ML measurement, and depth of the implant/test implant. It should be noted that the offset values of the implant/test implant may be used in combination with known geometrical ratios of the articular surface for a particular region of the articular surface. These geometric ratios may be found in published literature and may be utilized, for example, when the implant is placed proximate the interface between the posterior and distal regions of the articular surface. If further accuracy is desired (for example, but not limited to, defects extending further towards the posterior region and/or the anterior regions of the articular surfaces), the contour of the implant and articular surface may be determined as described in U.S. Patent No. 8, 177, 841 entitled System and Method for Joint Resurface Repair.

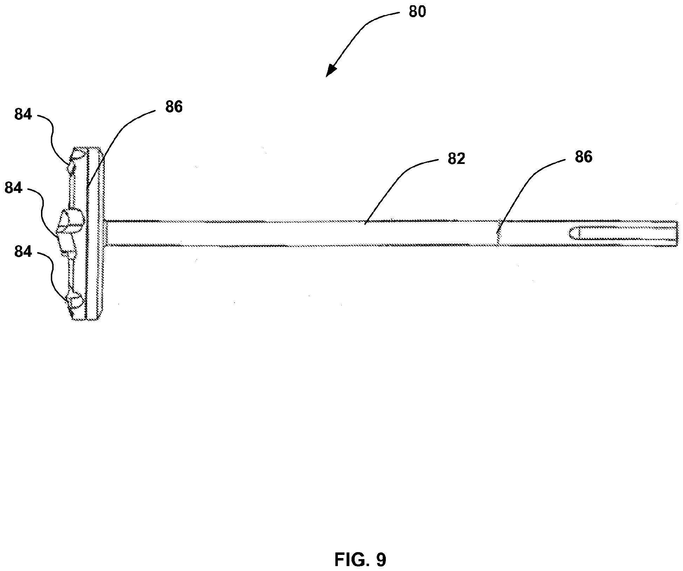

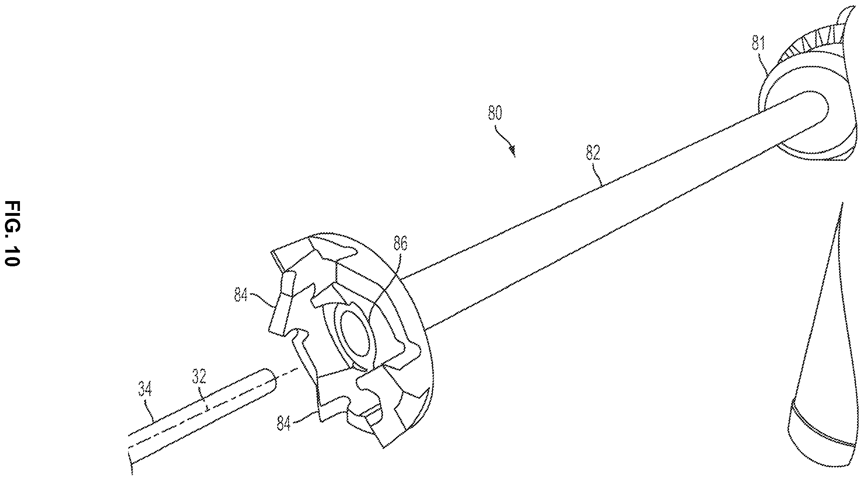

Turning now to **FIGS. 9-10****,** the diameter of a surface reamer 80 may be selected based on, for example, the maximum ML value. The surface reamer 80 may include a cannulated shaft 82 configured to be disposed over the guide pin 34 along the reference axis and coupled to a drill 81. The surface reamer 80 may also include one or more cutting surfaces 84. The reamer 80 may have a specific geometry or pattern to minimize vibrations and improve tactile feel while negotiating an interrupted cut on the trochlear groove.

The surface reamer 80 may be advanced over the guide pin 34 along the reference axis. The surface reamer 80 may include an indicia 86 (for example, an opening/window, laser marker, or the like) configured to control the depth of the bore B formed in the saddle or base 15 of the trochlear region. For example, the indicia 86 may include a laser marking or the like configured to be aligned with the articular surface 16. The indicia 86 may also include an opening/window or the like which may be aligned with an indicia on the guide pin. The cutters 84 may optionally be positioned about the surface reamer 80 to leave more material proximate the guide pin 34 along the reference axis to facilitate removal and insertion of devices further along the method. Once the articular surface 16 has been excised about the reference axis, the surface reamer 80 and the guide pin 34 may be removed.

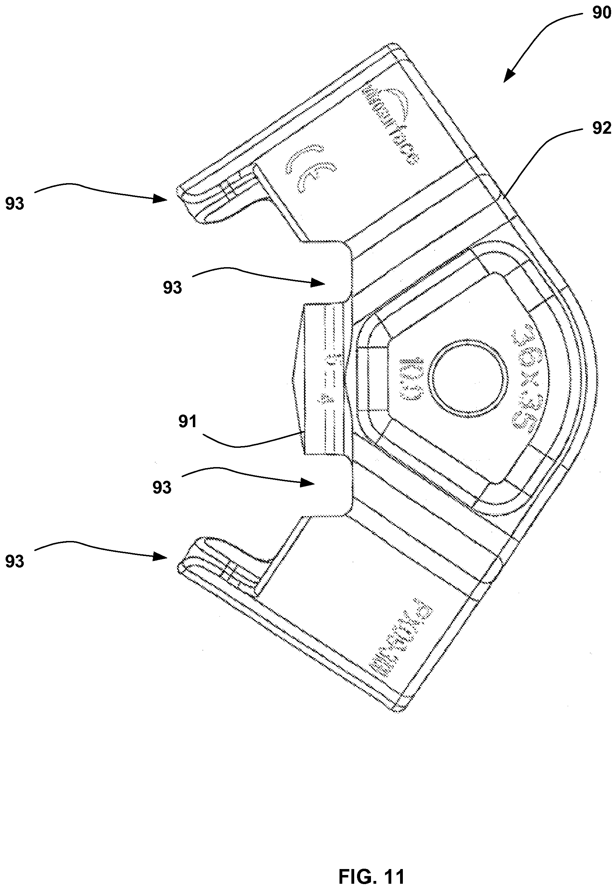

A guide block 90, **FIG. 11****,** may be selected based on the measurements taken previously of the patient's articular surface 16. The guide block 90 may be used to establish one or more working axis (for example, a superior and inferior working axis) for excising the articular surface 16 on either side of the reference axis along the superior-inferior plane. The guide block 90 may include a body 92 having a generally arcuate shaped exterior surface generally configured to engage with the base or saddle 15 and ridges 17a, 17b of the trochlear region 16. For example, a portion of the guide block 90 have an outer surface which is substantially the inverse of the articular surface 16 which is to be replaced in the trochlear region proximate the defect site 14.

The guide block 90 may further comprise a protrusion or tab 91 extending generally outwardly from the bottom or base surface 93 of the body 92. The protrusion 91 may be configured to be received in the bore B formed by the excision device in the articular surface 16 discussed above. As may be appreciated, the bore B may be formed in the base or saddle 15 of the trochlear region 16. According to at least one embodiment, the protrusion 91 and the bore B may have form a generally interference-like fit such that movement of the guide block 90 may be minimized when the protrusion 91 is received in the bore B.

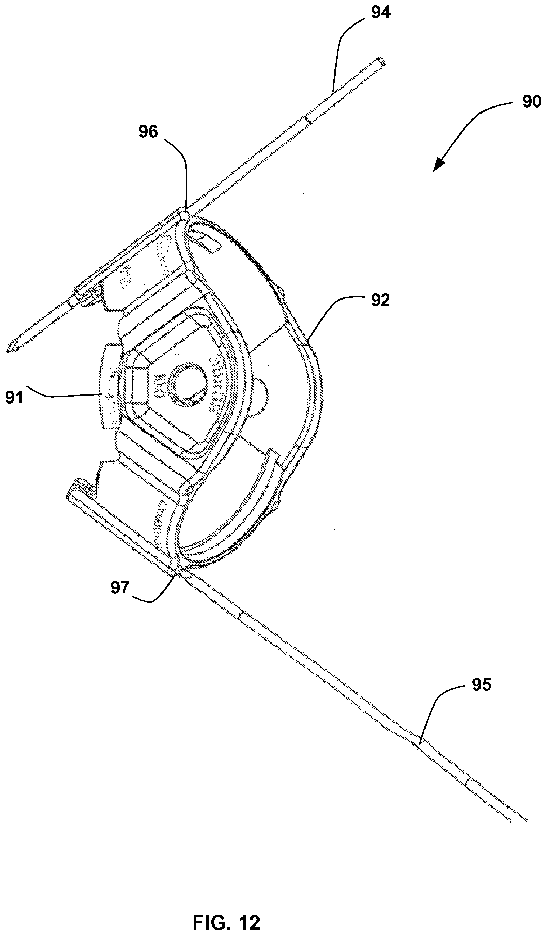

Turning now to **FIG. 12****,** the guide block 90 may also include one or more securing pins 94, 95 configured to further reduce movement of the guide block 90 relative to the articular surface 16. The pins 94, 95 may be configured to extend through passageways 96, 97 in the body 92 and may be secured (for example, but not limited to, screwed) into the knee. The pins 94, 95 may optionally be secured into the knee in regions which are generally not involved in the articulation of the patellar.

As may be appreciated, the position of the guide block 90 may be generally fixed relative to the articular surface 16 by virtue of the protrusion 91 received in the bore B formed in the articular surface 16, the pins 94, 95, and/or the outer surface configuration of the body 92 generally contacting the trochlear groove.

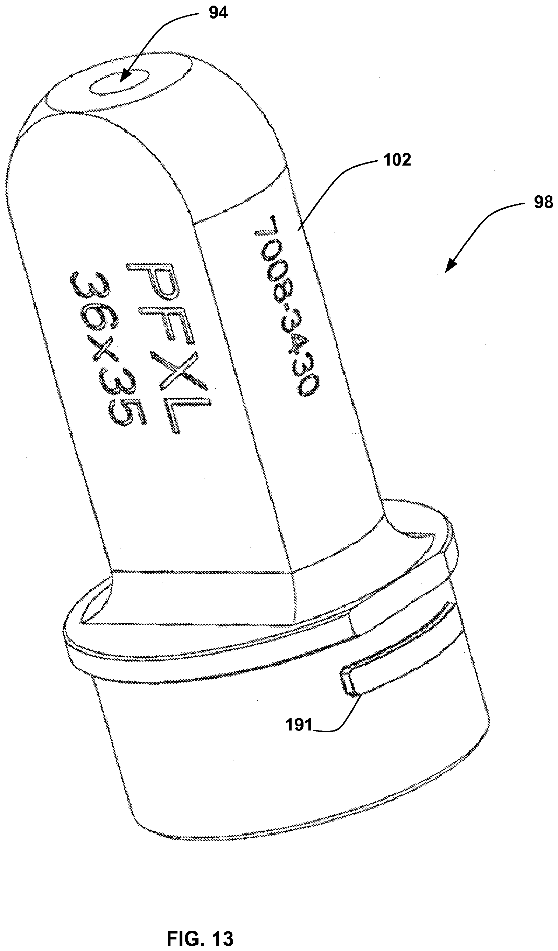

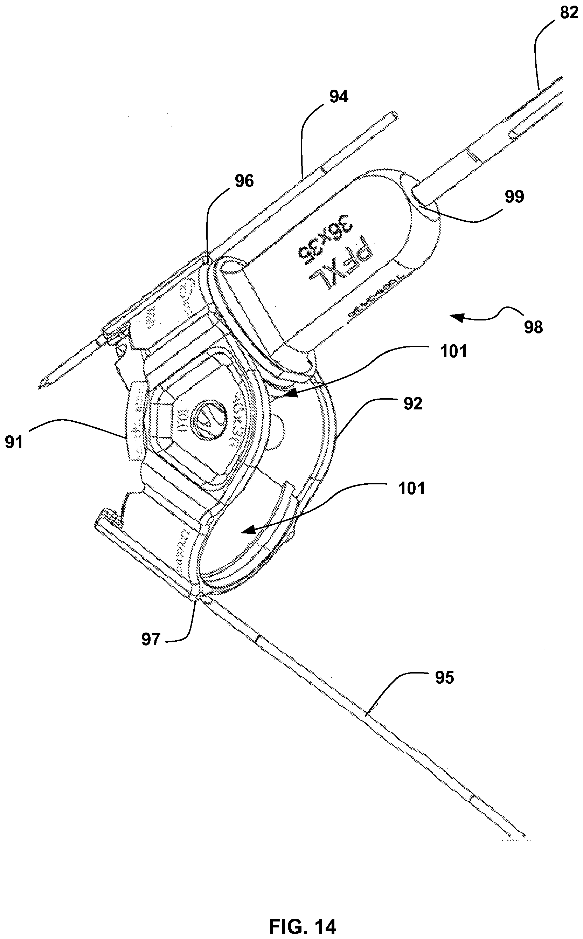

With the guide block 90 fixed/secured to the articular surface 16, additional excision sites may be formed for receiving the implant. For example, one or more guide bushings 98 may be used as generally illustrated in **FIG. 13****.** The guide bushing 98 may include a passageway 99 configured to receive the shaft 82 of the excision device 80. The guide bushing 98 may be configured to receive the shaft 82 such that the cutters 84 are disposed proximate the distal region 100 of the guide bushing 98. The distal region 100 of the guide bushing 98 may also be configured to be received in a cavity 101 formed in the guide block 90 as generally illustrated in **FIG. 14****.**

According to at least one embodiment consistent herein, the cavity 101 and the distal region 100 of the guide bushing 98 maybe configured to threadably engage each other. Alternatively, the cavity 101 and the distal region 100 of the guide bushing 98 may fit together in a generally interference-type connection. While the cavity 101 and the distal region 100 of the guide bushing 98 are illustrated having a generally circular or cylindrical cross-section, the cavity 101 and the distal region 100 of the guide bushing 98 may also include other cross-sectional shapes. For example, the cavity 101 and the distal region 100 of the guide bushing 98 may include a non-circular cross-sectional shape configured to generally prevent movement (rotational and/or translational) movement relative to each other. The guide bushing 98 may optionally include a handle portion 102 configured to facilitate coupling and decoupling of the guide bushing 98 with the cavity 101.

The guide block 90 may also include an opening configured to allow the cutter 80 to pass through the guide block 90 and into the articular surface 16 to form additional excision sites corresponding to the implant to be delivered. When received within the guide block 90, the guide bushing 98 may generally align the longitudinal axis L of the cutter 80 with the articular surface 16 at a predetermined angle relative to the working axis defined by the guide pin. The guide bushing 98 may generally minimize movement of the cutter 80 in any direction except along the predetermined angle with respect to the working axis.

According to at least one embodiment consistent herein, the guide block 90 may be configured to create at least one excision site partially overlapping with the primary excision site (i.e., the excision site corresponding to bore B). As illustrated in **FIG. 14****,** the guide block 90 is shown configured to receive a first and second guide bushing 98 (which may be the same or different) and may form a first and second additional excision site (each partially overlapping with the primary excision site bore B). The guide block 90 may, however, be configured to receive fewer or greater than two guide bushings 98 depending on the size and shape of the implant to be delivered as well as the particulars of the patient's anatomy. In addition, one or more of the additional excision sites formed with the guide block 90 may overlap only an adjacent additional excision site (i.e., one or more of the additional excision sites may not overlap with the primary excision site).

Once the excision sites are formed in the patient's articular surface 16, an implant sizing trial may be selected based on the measurements taken of the articular surface 16. The implant sizing trial may comprise a shape/contour generally corresponding to the shape/contour of the implant to be delivered. The implant sizing trial may comprise a threaded opening configured to be concentrically disposed about the working axis. The threaded opening may also be configured to be threadably engaged with a cannulated shaft/handle. Once the implant sizing trial is inserted into the excision sites in the articular surface 16, the fitment of the implant sizing trial along the inferior-superior and ML planes may be confirmed visually.

With the implant sizing trial inserted within the excision sites and the fitment confirmed, a cannulated pilot drill may be advanced through the handle and the implant sizing trial into the bone along the reference axis. The pilot drill may also include a depth control device such as, but not limited to, a marking (e.g., a laser marking or the like). With the cannulated pilot drill secured in the bone, the implant sizing trial and handle may be removed and the guide pin may be advanced through the cannulated passageway of the pilot drill into the bone along the reference axis. Again, the depth of the guide pin may be controlled by way of a marking (e.g., a laser marking or the like) along the shaft of the guide pin. For example, the depth of the guide pin may be set once the laser marking is flush with the end of the pilot drill.

A cannulated step drill may be advanced over the pilot drill and the guide pin into the articular surface 16 about the reference axis. The use of the pilot drill and the cannulated step drill may be configured to incrementally provide a larger opening in the bone about the reference axis in the articular surface 16 to reduce the potential of chipping the bone about the reference axis. The cannulated step drill may also include a depth stop for controlling the depth of the step drill into the bone.

Once the depth of the step drill is set, the step drill and the pilot drill may be removed and a cannulated tap may be advanced over the guide pin. The depth that the tap is advanced into the bone may be controlled based on a marking (e.g., a laser marking) on the guide pin. The tap may be configured to provide a threaded opening in the bone about the reference axis to threadably receive the implant post as will be described below.

With the opening about the reference axis tapped, the tap may be removed and a tapered post may be advanced over the guide pin at least partially into the threaded opening, for example, using a hex driver or the like. The tapered post may include a tapered and threaded first end and a second end having a tapered exterior surface, for example, as described in U.S. Patent Nos. 6,520,964, 6,610,067 and 6,679,917. The second end may also include a hex-shaped internal cavity configured to engage with a corresponding hex-shaped driver of the hex driver. Both the tapered post and the hex driver may be cannulated such that they may be advanced over the guide pin.

The tapered post may be advanced along the guide pin and partially inserted into the threaded opening in the bone (for example, approximately half way) using the hex driver. According to one embodiment, the tapered post may be inserted in the threaded opening such at least most of the threaded end is within the threaded opening. Once the tapered post is partially received in the threaded opening, the hex driver may be removed

The implant sizing trial may optionally be placed into the excision sites. The second end of the tapered post may at least partially extend through the threaded opening of the implant sizing trial. Using the hex driver, the implant sizing trial may be fully advanced into the threaded opening. The hex driver may include a flared end which may engage a shoulder disposed about the opening in the implant sizing trial. The engagement of the flared end and the shoulder may control the final depth of the tapered post into the threaded opening in the bone.

Once the tapered post is fully advanced into the threaded opening, the hex driver and implant sizing trial may be removed. Optionally, a cannulated reamer may be advanced over the guide pin to remove any excess material about the reference axis. The depth of the reaming may be controlled when the shoulder of the reamer contacts the end of the tapered post. The reaming may be provided to extra material left about the reference axis during the reaming discussed above. This extra material may have been left to prevent accidental chipping during the subsequent operations. After the final reaming, the reamer and the guide pin may be removed leaving behind only the tapered post in the bone.

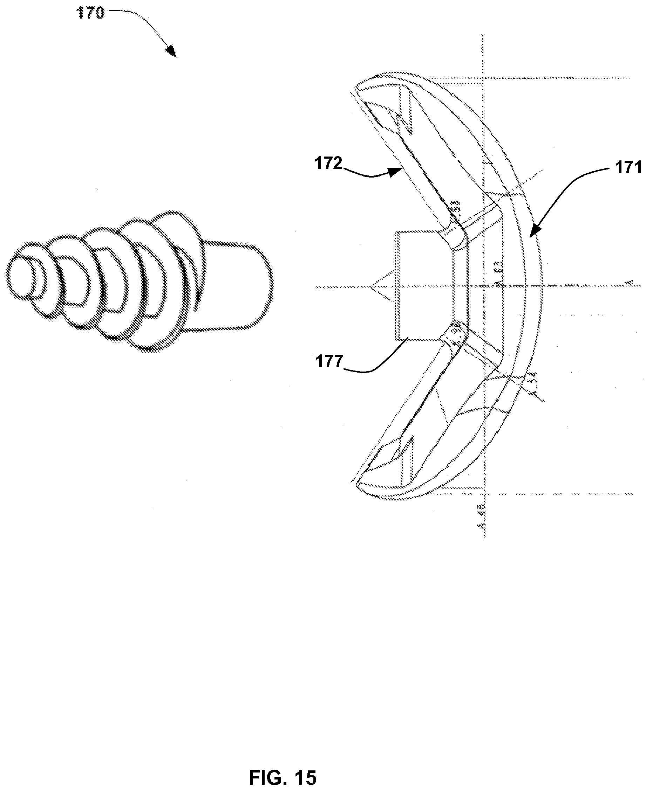

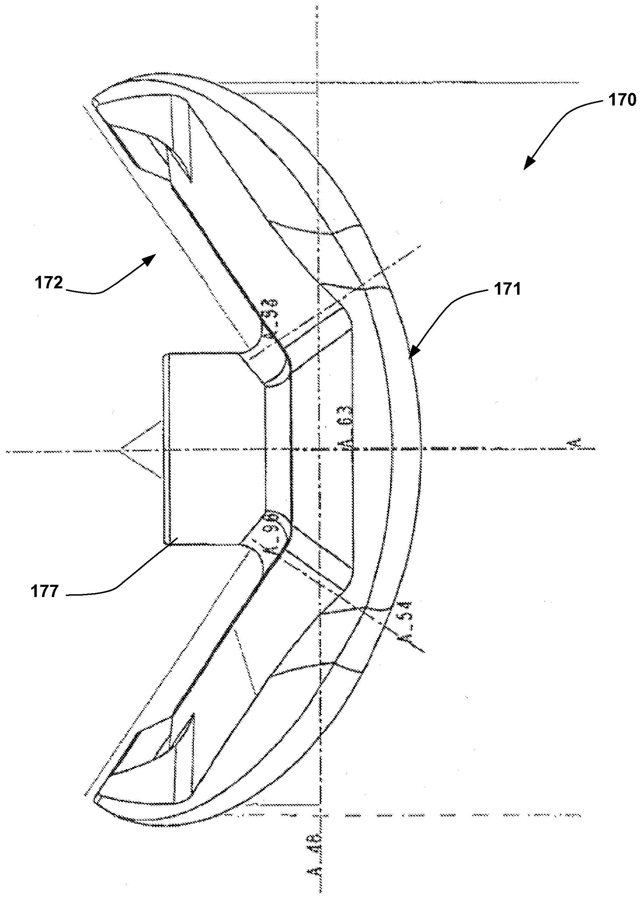

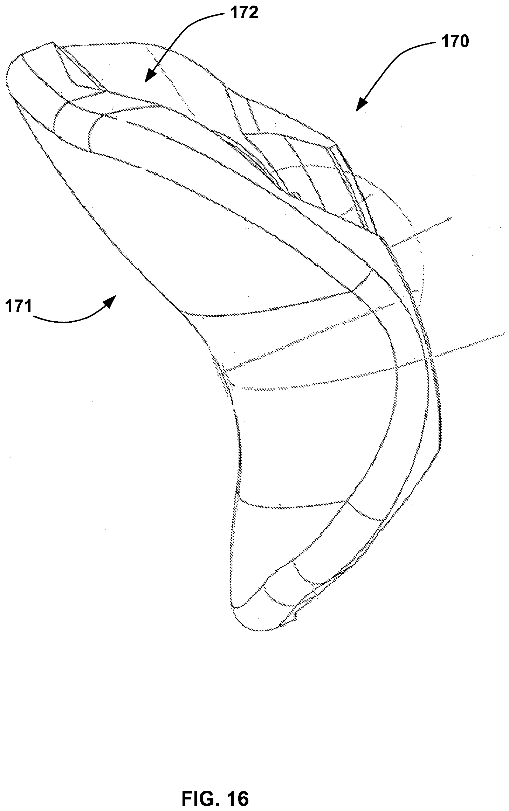

An implant 170, **FIGS. 15-18****,** may be selected base on the measurements taken of the patient's articular surface 16. As discussed previously, the implant 170 may have a load bearing surface 171 including a contour based on the measurements taken of the patient's articular surface 16 such that the load bearing surface 171 generally corresponds to the patient's original articular surface 16, for example, as best illustrated in **FIG. 17****.** In particular, the load bearing surface 171 may include a first curvature 181 (that may include multiple curves) based on or corresponding to the curvature of the articular surface 16 being replaced along the inferior-superior plane in base or saddle portion 15 of trochlear region. The load bearing surface 171 may also include a second curvature 182 (that may include multiple curves) based on or corresponding to the curvature of the articular surface 16 being replaced along the ML plane in ridge 17a, 17b portion of trochlear region. The second curvature 182 may include a curve string generally perpendicular to and swept along the length of the first curvature 181 and may vary along the length of the first curvature 181.

According to one embodiment, the implant 170 may include an implant as described in WO2008098061, U.S. Patent No. 6,679,917 issued January 20, 2004, U.S. Patent No. 6,610,067 issued August 26, 2003, U.S. Patent No. 6,520,964 issued February 18, 2003.

The bone facing surface 172 of the implant 170 may a plurality of regions revolved about the plurality of axis established by the guide pin and/or the guide block 90. For example, the bone facing surface 172 may include a contour substantially corresponding to the contour of the plurality of excision sites created in the patient's bone. Because these excisions sites may be created by a rotary cutter moving along the axes established by the guide pin and/or the guide block 90 (e.g., generally normal to the articular surface), the contours of the excision sites may be different than a planar cut (i.e., an excision site created by making a planar or tangential cut across the articular surface). The bone facing surface 172 may optionally include indicia 176 representing either inferior and/or superior sides of the implant 170 as well as the size of the implant 170. These indicia 176 may be used by the surgeon to properly align the implant 170 along the inferior-superior and ML planes within the excision sites. The implant 170 may be inserted into the excision site using a grasping device such as, but not limited to; a suction cup coupled to a handle.

The implant 170 may include a first fixation device 177 coupled to the bone facing surface 172. The first fixation device 177 may be configured to be received in the bore B formed in the articular surface 16. The first fixation device 177 may optionally be configured to engage with a second fixation element configured to be secured into the patient's bone.

For example, the second fixation element may include a post. The post may include a tapered cross-section and may optionally include a threaded outer region configured to engage with the patient's bone as discussed herein. The post may also include one more protrusion or flanges configured to engage with the patient's bone. The first and second fixation element may be configured to be coupled to each other as discussed in WO2008098061 U.S. Patent No. 6,679,917 issued January 20, 2004, U.S. Patent No. 6,610,067 issued August 26,2003, U.S. Patent No. 6,520,964 issued February 18, 2003. The first fixation device 177 of the implant 170 may include a female opening 185 configured to frictionally engage with a tapered second end of the tapered post.

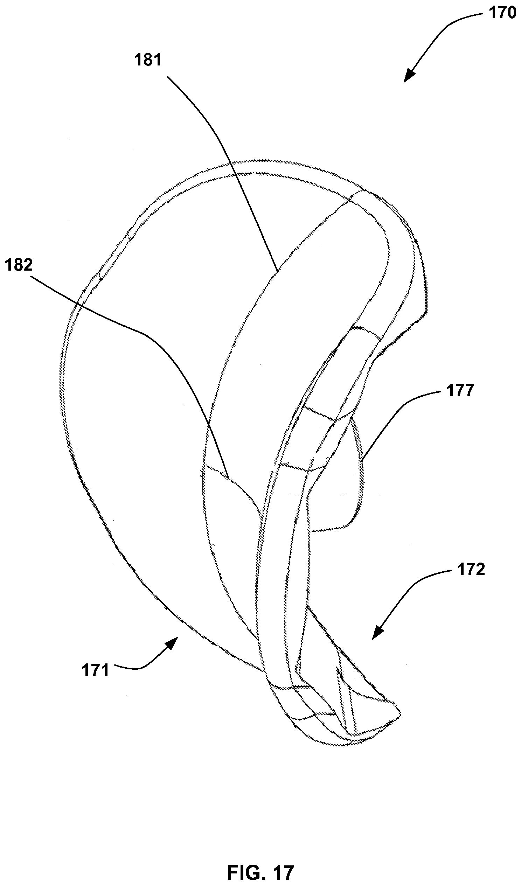

The bone facing surface 172 may also optionally include one or more rims, ribs or protrusions 180 extending generally downwardly and away from the bone facing surface 172, for example, as illustrated in **FIG. 18****.** For example, the rims 180 may include a superior rim 180a disposed proximate the superior end region 181 of the implant 170 and/or an inferior rim 180b disposed proximate the inferior end region 182 of the implant 170. The excisions sites corresponding to the rims 180 may be include a contour configured to receive the rims 180 (which may be formed by the excision cutter 80 and/or may be formed separately).

An adhesive (such as, but not limited to, bone cement or the like) may be applied to the bone facing surface 172 by way of a dispenser, for example a dispenser as described in WO2008101097. The female opening 185 of the implant 170 may receive and frictionally engage with the tapered second end of the tapered post. For example, the implant 170 may be mated in the excision sites and to the tapered post using an impactor and hammer.

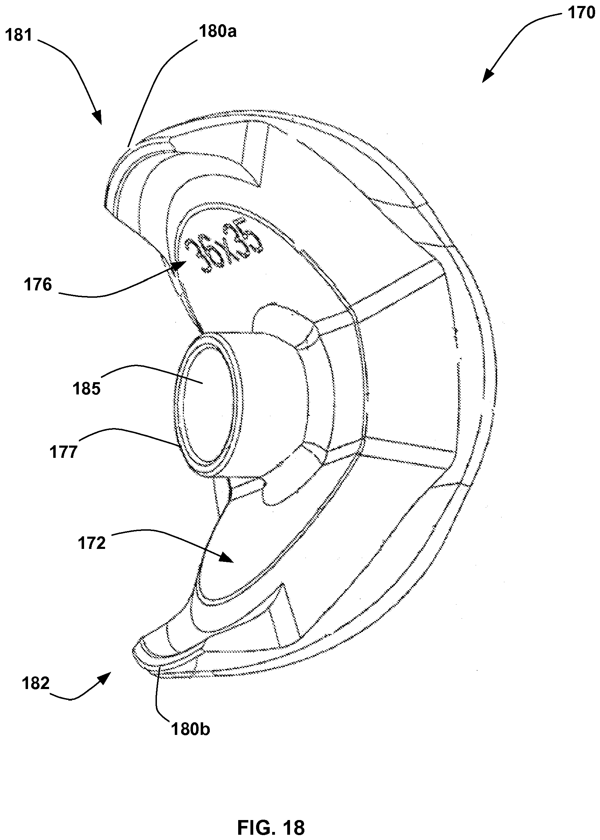

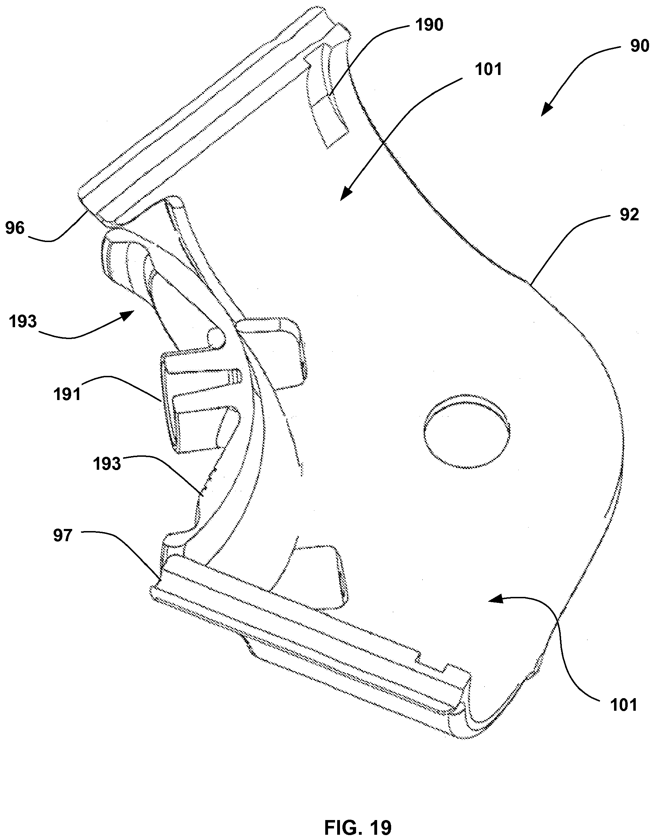

Turning now to **FIG. 19****,** a cross-sectional view of one embodiment of a guide block 90 is illustrated. As may be seen, the guide block 90 may include one or more cavities 101 configured to receive the guide bushings 98. For example, the cavities 101 may include a threaded region 190 configured to engage with a corresponding threaded region 191 of the guide bushings 98 (for example, the threaded region 191 illustrated in **FIG. 13**). The guide block 90 may also include one or more openings or apertures 193 configured to allow the cutting head of the excision device 80 to pass through the guide block 90 and into the articular surface below the guide block 90.

According to one aspect, the present disclosure may feature a system for repairing a defect on an articular surface of a patient's trochlear region. The system may comprise a guide block comprising a body having an exterior surface configured to engage with the saddle portion and ridge portions of the patient's trochlear region. A protrusion may extend generally from the body and may be configured to be received in a first bore formed in the articular surface along a reference axis. A first cavity may extend through the body configured to establish a first working axis displaced from the reference axis. The exterior surface of the body and the protrusion may be configured to secure the location of the guide block about the patient's trochlear region.

According to another aspect, the present disclosure may feature a method for preparing an implant site in bone, comprising: establishing a reference axis extending from the bone; creating a bore in the bone by reaming about the reference axis; securing a guide block about the articular surface; establishing a first working axis extending from the bone using the guide block, the first working axis is displaced from the reference axis; and creating a first socket in the bone by reaming about the first working axis, wherein the first socket partially overlaps with the bore.

As mentioned above, the present disclosure is not intended to be limited to a system or method which must satisfy one or more of any stated or implied object or feature of the present disclosure and should not be limited to the preferred, exemplary, or primary embodiment(s) described herein. The foregoing description of a preferred embodiment of the present disclosure has been presented for purposes of illustration and description. It is not intended to be exhaustive or to limit the present disclosure to the precise form disclosed. Obvious modifications or variations are possible in light of the above teachings. The embodiment was chosen and described to provide the best illustration of the principles of the present disclosure and its practical application to thereby enable one of ordinary skill in the art to utilize the present disclosure in various embodiments and with various modifications as is suited to the particular use contemplated. All such modifications and variations are within the scope of the present disclosure.

## Claims

1. A system for repairing a defect on an articular surface of a patient's bone, said system comprising:
a reamer (80) comprising a shaft (82) and a cutting head (86) extending generally radially outward from an end thereof, said cutting head having at least one cutting surface (84); and
a guide block (90) comprising:
a body (92) having an exterior surface configured to engage against said articular surface of said bone;
a protrusion (91) extending generally from said body and configured to be received in a first bore formed in said articular surface along a reference axis; and
a first excision passageway (99) extending through a guide bushing (98) extending through a first cavity (101) in said body (92) along a first working axis,
**characterized in that** said first excision passageway is configured to receive said shaft (82) and allow said shaft to only move along said working axis;
that a first opening disposed within said exterior surface of said body and aligned with said first excision passageway, said first opening configured to receive said cutting head between said body and said articular surface and to allow said cutting head to pass through said guide block (90) and into the articular surface below the guide block (90) to form an excision site in said articular surface centered around said first working axis;
and that said reamer (80) comprises indicia on said shaft (82) configured to be aligned with said first excision passageway to define a depth of said excision site formed by said reamer in said articular surface.

2. The system of claim 1, wherein said body (92) includes a base portion and sidewall portions having a generally arcuate shaped exterior surface generally configured to engage with a saddle portion (15) and ridge portions (17a, 17b) of a patient's trochlear region (16), respectively.

3. The system of one of claims 1 or 2, further comprising a first pin (94) and a first pin passageway (96) extending through said body (92) configured to receive said first pin, wherein said first pin is configured to engage bone proximate to said articular surface, and wherein said exterior surface of said body, and said first pin extending through said first pin passageway are configured to secure the location of said guide block (90) about said patient's articular surface.

4. The system of claim 3, further comprising a second pin (95) and a second pin passageway (97) extending through said body (90) configured to receive said second pin, wherein said second pin is configured to engage bone proximate to said articular surface, and wherein said exterior surface of said body, and said first and said second pins extending through said first and second pin passageways (96, 97) are configured to secure the location of said guide block about said patient's articular surface.

5. The system of one of claims 1 to 4, wherein said protrusion (91) is configured to be received in said first bore in a generally interference-type fit.

6. The system of one of claims 1 to 5, wherein said first guide bushing (98) is configured to be removably received in a first cavity (101) formed in said body, said first guide bushing defining said first excision passageway (99) generally aligned with said first working axis.

7. The system of claim 6, wherein said first guide bushing (98) is configured to threadably engage said first cavity.

8. The system of claim 1, wherein said first excision passageway (99) is configured to receive said shaft (82) of said excision device such that said excision site in said articular surface partially overlaps with a first bore formed in said articular surface.

9. The system of one of claims 1 to 8, further comprising a second excision passageway extending through a second guide bushing extending in said body configured to establish a second working axis displaced from said reference axis, wherein said second working axis is configured to define a center point of a third bore in said articular surface.

10. The system of claim 9, wherein said first and second excision passageways are configured to be generally aligned along an inferior-superior plane of said articular surface.

11. The system of claim 1, wherein said indicia (86) include a marking.

12. The system of claim 1, wherein said indicia (86) include a window.

13. The system of claim 6, wherein said cutting head is configured to be disposed within said first opening while said first guide bushing is received in said first cavity such that said cutting head is disposed proximate to a distal region of said first guide bushing.

14. The system of claim 1, further comprising an implant (170) configured to be at least partially received in said excision site.

15. The system of claim 14, wherein said implant (170) includes a load bearing surface including a contour based on said patient's articular surface, and a bone facing surfacing revolved around said first working axis.

16. The system of claim 15, wherein said bone facing surface (172) has a contour substantially corresponding to a contour of said excision site.

17. The system of claim 16, wherein said bone facing surface (172) includes a first fixation element (177) configured to engage a second fixation element, said second fixation element configured to be secured in said bone beneath said excision site.

## Patentansprüche

1. System zum Reparieren eines Schadens an einer Gelenkfläche eines Knochens eines Patienten, wobei das System umfasst:
eine Reibahle mit einer Welle (82) und einem Schneidkopf (86), der sich von einem Ende derselben allgemein radial nach außen erstreckt, wobei der Schneidkopf mindestens eine Schneidfläche (84) aufweist; und
einen Führungsblock (90), umfassend:
einen Körper (92) mit einer Außenfläche, die derart konfiguriert ist, dass sie an der Gelenkfläche des Knochens angreift;
einen Vorsprung (91), der sich allgemein von dem Körper erstreckt und konfiguriert ist für die Aufnahme in einer ersten Bohrung, die entlang einer Referenzachse in der Gelenkfläche gebildet ist; und
einem ersten Exzisionskanal (99), der durch eine Führungshülse (98) verläuft, die sich entlang einer ersten Arbeitsachse durch einen ersten Hohlraum (101) in dem Körper (92) erstreckt,
**dadurch gekennzeichnet,**
**dass** der erste Exzisionskanal konfiguriert ist für die Aufnahme der Welle (82) und eine Bewegung der Welle nur entlang der Arbeitsachse zulässt, dass in der Außenfläche des Körpers eine erste Öffnung angeordnet und auf den ersten Exzisionskanal ausgerichtet ist, wobei die Öffnung konfiguriert ist für die Aufnahme des Schneidkopfes zwischen dem Körper und der Gelenkfläche und ermöglicht, dass sich der Schneidkopf durch den Führungsblock (90) hindurch und in die Gelenkfläche unter dem Führungsblock (90) hinein bewegt, um in der Gelenkfläche eine Exzisionsstelle zu bilden, die um die erste Arbeitsachse zentriert ist;
und **dass** die Reibahle (80) an der Welle (82) Indizia aufweist, die für eine Ausrichtung mit dem ersten Exzisionskanal konfiguriert sind, um eine Tiefe der Exzisionsstelle zu definieren, die durch die Reibahle in der Gelenkfläche gebildet wird.

2. System nach Anspruch 1, wobei der Körper (92) einen Basisbereich und Seitenwandbereiche umfasst, die eine allgemein bogenförmige Außenfläche aufweisen, die allgemein für den jeweiligen Eingriff mit einem Sattelbereich (15) und mit Kammbereichen (17a, 17b) einer Trochlea-Region (16) eines Patienten konfiguriert ist.

3. System nach einem der Ansprüche 1 oder 2, ferner umfassend einen ersten Pin (94) und einen ersten Pin-Kanal (90), der sich durch den Körper (92) erstreckt und konfiguriert ist für die Aufnahme des ersten Pin, wobei der erste Pin ausgebildet ist für den Eingriff in Knochen in der Nähe der Gelenkfläche und wobei die Außenfläche des Körpers und der sich durch den ersten Pin-Kanal erstreckende erste Pin ausgebildet sind für die Lagesicherung des Führungsblocks (90) um die Gelenkfläche des Patienten.

4. System nach Anspruch 3, ferner umfassend einen zweiten Pin (95) und einen zweiten Pin-Kanal (97), der sich durch den Körper (90) erstreckt und konfiguriert ist für die Aufnahme des zweiten Pin, wobei der zweite Pin ausgebildet ist für den Eingriff in Knochen in der Nähe der Gelenkfläche und wobei die Außenfläche des Körpers und der sich durch den ersten und den zweiten Pin-Kanal (96, 97) erstreckende erste und zweite Pin ausgebildet sind für die Lagesicherung des Führungsblocks um die Gelenkfläche des Patienten.

5. System nach einem der Ansprüche 1 bis 4, wobei der Vorsprung (91) konfiguriert ist für die Aufnahme allgemein als Presspassung in der ersten Bohrung.

6. System nach einem der Ansprüche 1 bis 5, wobei die erste Führungshülse (98) konfiguriert für die entfernbare Aufnahme in einem in dem Körper gebildeten ersten Hohlraum (101), wobei die erste Führungshülse den ersten Exzisionskanal (99) definiert, der allgemein auf die erste Arbeitsachse ausgerichtet ist.

7. System nach Anspruch 6, wobei die erste Führungshülse (98) konfiguriert ist für den Gewindeeingriff in dem ersten Hohlraum.

8. System nach Anspruch 1, wobei der erste Exzisionskanal (99) konfiguriert ist für die Aufnahme der Welle (82) des Exzisionsgeräts, so dass sich die Exzisionsstelle in der Gelenkfläche mit der in der Gelenkfläche gebildeten ersten Bohrung teilweise überlappt.

9. System nach einem der Ansprüche 1 bis 8, ferner umfassend einen zweiten Exzisionskanal, der durch eine zweite Führungshülse verläuft, die sich in dem Körper erstreckt, und konfiguriert ist für die Bildung einer zweiten Arbeitsachse, die von der Referenzachse versetzt ist, wobei die zweite Arbeitsachse derart konfiguriert ist, dass diese einen Mittelpunkt einer dritten Bohrung in der Gelenkfläche definiert.

10. System nach Anspruch 9, wobei der erste und der zweite Exzisionskanal derart konfiguriert sind, dass die Exzisionskanäle allgemein entlang einer Inferior-Superior-Ebene der Gelenkfläche ausgerichtet sind.

11. System nach Anspruch 1, wobei die Indizia (86) eine Markierung umfassen.

12. System nach Anspruch 1, wobei die Indizia (86) ein Fenster umfassen.

13. System nach Anspruch 6, wobei der Schneidkopf konfiguriert ist für eine Anordnung in der ersten Öffnung, während die erste Führungshülse in dem ersten Hohlraum derart aufgenommen wird, dass der Schneidkopf in der Nähe eines distalen Bereichs der ersten Führungshülse angeordnet ist.

14. System nach Anspruch 1, ferner umfassend ein Implantat (170), das zumindest für die teilweise Aufnahme in der Exzisionsstelle konfiguriert ist.

15. System nach Anspruch 14, wobei das Implantat (170) eine Lastaufnahmefläche mit einer auf der Gelenkfläche des Patienten basierenden Kontur und einen dem Knochen zugewandten Belag umfasst, der um die erste Arbeitsachse gedreht wird.

16. System nach Anspruch 15, wobei die dem Knochen zugewandte Fläche (172) eine Kontur hat, die im Wesentlichen einer Kontur der Exzisionsstelle entspricht.

17. System nach Anspruch 16, wobei die dem Knochen zugewandte Fläche (172) ein erstes Befestigungselement (177) aufweist, das für einen Eingriff mit einem zweiten Befestigungselement konfiguriert ist, wobei das zweite Befestigungselement für eine Festlegung in dem Knochen unter der Exzisionsstelle konfiguriert ist.

## Revendications

1. Système pour réparer un défaut sur une surface articulaire d'un os de patient, le système comprenant:
un aléseur (80) avec un arbre (82) et une tête de coupe (86) s'étendant généralement vers l'extérieur à partir d'une extrémité de ce dernier, la tête de coupe présentant au moins une surface de coupe (84); et
un bloc de guidage (90) comprenant:
un corps (92) avec une surface extérieure configurée pour s'engager contre ladite surface articulaire de l'os;
une protubérance (91) s'étendant généralement à partir dudit corps et configurée pour être reçue dans un premier alésage formé dans ladite surface articulaire le long d'un axe de référence; et
un premier passage d'excision (99) s'étendant à travers une douille de guidage (98) s'étendant à travers une première cavité (101) dans ledit corps (92) le long un premier axe de travail,
**caractérisé en ce que** le premier passage d'excision est configuré pour recevoir l'arbre (82) et pour permettre le mouvement dudit arbre seulement le long de l'axe de travail;
qu'une première ouverture est disposée dans la surface extérieure du corps et alignée avec le premier passage d'excision, ladite première ouverture étant configurée pour recevoir la tête de coupe entre le corps et la surface articulaire et pour permette le passage de la tête de coupe à travers le bloc de guidage (90) et dans la surface articulaire en dessous du bloc de guidage (90), pour former un site d'excision dans la surface articulaire centrée autour dudit premier axe de travail; et
que ledit aléseur (80) comprend des indices sur ledit arbre (82) configurés pour être alignés avec le premier passage d'excision pour définir une profondeur du site d'excision formée par l'aléseur dans la surface articulaire.

2. Système selon la revendication 1, dans lequel le corps (92) comprend und partie de base et des parties de paroi latérale présentant une surface extérieure généralement de forme arquée et généralement configurée pour venir en prise avec une partie en forme de selle (15) and avec des parties d'arêtes (17a, 17b) d'une région de trochlée (16) d'un patient.

3. Système selon l'une des revendications 1 ou 2, en outre comprenant un premier broche (94) et un premier passage de broche (96) s'étendant à travers le corps (92) configuré pour recevoir le premier broche, ledit premier broche étant configuré pour venir en prise avec un os à proximité de la surface articulaire, et la surface extérieure du corps et le premier broche, qui s'étend á travers le premier passage de broche, étant configurés pour fixer l'emplacement du bloc de guidage (90) autour la surface articulaire du patient.

4. Système selon la revendication 3, en outre comprenant un deuxième broche (95) et un deuxième passage de broche (97) s'étendant à travers le corps (90) configuré pour recevoir le deuxième broche, le deuxième broche étant configuré pour venir en prise avec un os à proximité de la surface articulaire, et la surface extérieure du corps et le premier broche et le deuxième broche, qui s'étendent à travers le premier et le deuxième passage de broche (96, 97), étant configurés pour fixer l'emplacement du bloc de guidage autour la surface articulaire du patient.

5. Système selon l'une des revendications 1 à 4, dans lequel la protubérance (91) est configurée pour être reçue dans le premier alésage généralement à la manière d'un ajustement serré.

6. Système selon l'une des revendications 1 à 5, dans lequel la première douille de guidage (98) est configurée pour être reçue de manière amovible dans une première cavité (101) formée dans le corps, la première douille de guidage définissant le premier passage d'excision (90) généralement aligné avec le premier axe de travail.

7. Système selon la revendication 6, dans lequel la première douille de guidage (98) est configurée pour s'engager par filetage dans la première cavité.

8. Système selon la revendication 1, dans lequel le premier passage d'excision (99) est configuré pour recevoir l'arbre (82) du dispositif d'excision de sorte que le site d'excision dans la surface articulaire recouvre partiellement un premier alésage formé dans la surface articulaire.

9. Système selon l'une des revendications 1 à 8, en outre comprenant un deuxième passage d'excision s'étendant à travers une deuxième douille de guidage s'étendant dans le corps configuré pour créer un deuxième axe de travail déplacé de l'axe de référence, le deuxième axe de travail étant configuré pour définir un point central d'un troisième alésage dans la surface articulaire.

10. Système selon la revendication 9, dans lequel les premier et deuxième passages d'excision sont configurés pour être généralement alignés le long un plan inférieur-supérieur de la surface articulée.

11. Système selon la revendication 1, dans lequel les indices (86) comprennent un marquage.

12. Système selon la revendication 1, dans lequel les indices (86) comprennent une fenêtre.

13. Système selon la revendication 6, dans lequel la tête de coupe est configurée pour être disposée dans la première ouverture, tandis que la première douille de guidage est reçue dans la première cavité de sorte que la tête de coupe est disposée à proximité d'une région distale de la première douille de guidage.

14. Système selon la revendication 1, en outre comprenant un implant (170) configuré pour être reçu dans le site d'excision au moins partiellement.

15. Système selon la revendication 14, dans lequel l'implant (170) comprend une surface portante avec un contour basé sur la surface articulaire du patient, et un surfaçage faisant face à l'os et tourné autour le premier axe de travail.

16. Système selon la revendication 15, dans lequel la surface opposée à l'os (172) présente un contour qui correspond sensiblement au contour du site d'excision.

17. Système selon la revendication 16, dans lequel la surface faisant face à l'os (172) comprend un premier élément de fixation (177) configuré pour venir en prise à un deuxième élément de fixation, ledit deuxième élément de fixation étant configuré pour être fixé dans l'os en dessous du site d'excision.
